# EUROPÄISCHE PATENTSCHRIFT

(11) **EP 3 251 724 B1**
(45) Veröffentlichungstag und Bekanntmachung des Hinweises auf die Patenterteilung: **02.01.2019**
(21) Anmeldenummer: 17170832.4
(22) Anmeldetag: 12.05.2017
(51) Int. Cl.: A61N 1/375

(54) **DURCHFÜHRUNGSBAUTEIL MIT STRUKTURIERTER METALLBESCHICHTUNG UND VERFAHREN ZUR HERSTELLUNG EINES SOLCHEN DURCHFÜHRUNGSBAUTEILS**
FEEDTHROUGH COMPONENT WITH STRUCTURED METAL COATING AND METHOD FOR PRODUCING SUCH A FEEDTHROUGH COMPONENT
ÉLÉMENT CONSTITUTIF COMPRENANT UN REVÊTEMENT MÉTALLIQUE STRUCTURÉ ET SON PROCÉDÉ DE FABRICATION

(30) Priorität: 30.05.2016 DE 102016109898
(43) Veröffentlichungstag der Anmeldung: 06.12.2017
(73) Patentinhaber: BIOTRONIK SE & Co. KG, 12359 Berlin (DE)
(72) Erfinder: KRONMÜLLER, Daniel, 90409 Nürnberg (DE); KALB, Hermann, 91052 Erlangen (DE)
(74) Vertreter: Keck, Hans-Georg

(56) Entgegenhaltungen:
- DE-A1-102014 204 528
- US-A1- 2007 277 374
- US-A1- 2010 258 342
- US-A1- 2015 157 862
- US-A1- 2015 250 386

## Beschreibung

### Gebiet der Erfindung

Die Erfindung betrifft ein Verfahren zur Herstellung eines Durchführungsbauteils eines medizinelektronischen Gerätes, insbesondere eines implantierbaren Gerätes, wobei ein Durchführungs-Grundkörper, insbesondere aus Keramik, bereitgestellt und großflächig mit einer Metallbeschichtung versehen und die Metallbeschichtung anschließend strukturiert wird. Sie betrifft des Weiteren ein Durchführungsbauteil, das insbesondere mit einem solchen Verfahren hergestellt werden kann.

Medizinelektronische Geräte, die derartige Durchführungsbauteile enthalten, sind insbesondere als Herzschrittmacher, implantierbare Kardioverter (speziell Defibrillatoren) oder Cochleaimplantate seit langem in massenhaftem Einsatz. Es kann sich hierbei aber auch um eine weniger komplexe Vorrichtung, wie etwa eine Elektroden- oder Sensorleitung, handeln.

Die meisten praktisch bedeutsamen implantierbaren elektromedizinischen Geräte sind dazu vorgesehen, über geeignet platzierte Elektroden elektrische Impulse an reizbares Körpergewebe abzugeben. Ferner können viele Geräte Signale des Nervengewebes im Körper des Patienten gezielt messen und über einen längeren Zeitraum aufzeichnen oder auswerten, um eine individuell angepasste Therapie zu wählen und den Erfolg der Behandlung in vivo zu überprüfen. Um diese Funktion auszuführen, sind im Gehäuse des Gerätes elektronische/elektrische Funktionseinheiten zur Erzeugung und zum Regeln der Impulse und zum Messen von Reizen untergebracht. Außen am Gerät sind Elektroden oder Anschlüsse für mindestens eine Elektrodenleitung vorgesehen, in deren distalem Endabschnitt die Elektroden zur Impulsübertragung an das Gewebe angebracht sind.

Hierfür muss eine elektrische Verbindung zwischen den im Gehäuseinneren angeordneten elektrischen und/oder elektronischen Bauteilen und der jeweiligen Elektrodenleitung hergestellt werden. Diese elektrische Verbindung wird in der Regel mittels einer Durchführung und/oder einem sogenannten Header realisiert.

Die US 2010/0258342 A1, die alle Merkmale des Oberbegriffs der Ansprüche 1 und 12 offenbart, offenbart so eine Durchführung.

Eine derartige Durchführung sorgt dabei für mindestens eine elektrische Verbindung zwischen dem Inneren des Gehäuses und dem Äußeren und schließt gleichzeitig das Gehäuse des Implantats hermetisch ab. Der über der Durchführung befestigte Header führt die elektrische Verbindung der Durchführung weiter zu einer Kontaktstelle und dient zum Einstecken der mindestens einen Elektrodenleitung in eine entsprechende, meist genormte Buchse. An den Kontaktstellen der Buchse wird so ein elektrischer Kontakt zwischen dem Implantat und dem Anschlussstück der Elektrodenleitung hergestellt. Eine Durchführung und ein Header können auch in einem einzigen Bauteil realisiert sein. Auch in diesem Fall wird ein solches kombiniertes Bauteil im Folgenden allgemein als Durchführung bezeichnet.

Der Flansch, welcher zum hermetischen Abschluss des Gehäuses oder Implantates mit der Durchführung benötigt wird, besteht üblicherweise aus einem Metall (z. B. Titan) oder einer Legierung (z. B. Ti-6Al-4V). Vorteilhaft ist es, Flanschmaterial und Gehäuse- bzw. Implantatmaterial aus artgleichen Werkstoffen herzustellen um diese in Folgeprozessen leichter miteinander fügen zu können. Der Isolationskörper der Durchführung besteht im Wesentlichen aus einem schlecht leitenden Material (z. B. Isolationskeramik).

Die Kontaktelemente durchstoßen den Isolationskörper und werden durch den Isolationskörper elektrisch voneinander und gegen den Flansch elektrisch isoliert. Die Elemente bestehen üblicherweise aus gut leitenden Metallen (Tantal, Niob, Titan, Platin) oder Legierungen (PtIr, FeNi, 316L). Für die Herstellung von Kontaktelementen werden häufig Drahtabschnitte, sogenannte Stifte, verwendet.

Eine große Herausforderung ist es, die unterschiedlichen physikalischen Eigenschaften der Komponenten und Materialien, insbesondere die thermischen Ausdehnungskoeffizienten, so aufeinander abzustimmen, so dass für die Durchführung eine über die vorgesehene Lebensdauer ausreichende Dichtheit gewährleistet werden kann.

Bei der Herstellung von Keramikdurchführungen werden die Komponenten (Isolationskeramiken, Flansch, Stifte) mittels eines Hartlötprozesses (brazing process) gefügt. Zur Gewährleistung der Qualität der Lotverbindungen bei Durchführungen der genannten Art werden die jeweiligen Isolationskörper partiell mit einer metallischen Beschichtung versehen, die die Hartlötfähigkeit des Isolationskörpers sicherstellen soll. Bei der Beschichtung handelt es sich um ein elementares Metall (z. B. Wolfram, Niob, Titan) oder um eine Legierung. Die Beschichtung wird meist mittels eines PVD Prozesses auf der Isolationskeramik abgeschieden und hat eine typische Schichtdicke von 1-20 µm.

Es versteht sich, dass diese metallische Beschichtung lediglich bereichsweise vorhanden sein darf bzw. eine vordefinierte Struktur aufweisen muss, denn die Isolationskeramik der Durchführung trennt zwei Potentiale (einerseits das Massepotential am Flansch bzw. Gehäuse, andererseits das Signalpotential am Stift) auf engem Raum elektrisch voneinander. Die Trennung der Potentiale kann erreicht werden, indem man Beschichtung partiell wieder abträgt oder partiell keine Beschichtung aufbringt.

Während des Beschichtens muss eine ausreichend dicke, möglichst homogene Schicht gewährleistet werden. Jedoch gilt es zu verhindern, dass beim fertigen Durchführungsbauteil die erwarteten Potentiale kurzgeschlossen werden bzw. dass der Isolationswiderstand unzulässig verringert wird.

Wird eine auf die Isolationskeramik aufgebrachte Beschichtung mittels mechanischen Abtragens (Schleifen, Läppen) in Bereiche aufgetrennt, so müssen bereits durch den Konstrukteur der Isolationskeramik entsprechende Konturen bzw. Aufmaße vorgesehen werden. Während der Entschichtung ist die Isolationskeramik mechanischen Druck bzw. Zugbeanspruchung ausgesetzt, welche das Produkt belasten.

Alternativ kann die Beschichtung bereits beim Auftragen mittels Masken strukturiert werden. Der Maskierungsprozess stellt hohe Anforderungen an die Maßhaltigkeit der Isolationskeramik und Prozessfähigkeit des Beschichtungsprozesses. Um konstante Prozessergebnisse zu erhalten, müssen die Maskierungen und die Isolationskeramiken auch bei verschiedenen Eingangschargen und über mehrere Prozesse hinweg in Maß und Toleranz exakt zueinander passen. Durch das Beschichten verlieren die Masken ihre Maßhaltigkeit und müssen nachgearbeitet oder ausgewechselt werden.

Die Masken müssen sehr eng um die Isolationskeramiken aufliegen, da sonst Beschichtung auch hinter der Maske aufgebracht wird. Ein ungleichmäßiges Aufliegen führt zu einer ungleichmäßigen Beschichtungskante und damit zwangsläufig auch zu Streuungen der elektrischen Eigenschaften (Kapazität, Induktivität) der Isolationskeramiken und somit auch zu Serienstreuungen der Durchführung und des medizinischen Gerätes. Die während des Prozesses eingesetzten Masken werfen selbst Schatten und verringern so die Abscheiderate an den zu beschichtenden Isolationskeramiken. Ferner führen ungleichmäßige Übergänge zwischen Beschichtung und maskiertem Bereich zu optischen Auffälligkeiten am Produkt oder zu Problemen in Folgeprozessen und sind daher unerwünscht.

Das mechanische Bestücken bzw. Entstücken der Masken stellt sehr hohe Anforderungen an die Wiederholgenauigkeit von Handlingsystemen (Robotern, Linearachsen, Kamerasystemen). Bei unsachgemäßen Handling kann die Isolationskeramik oder deren Beschichtung zerstört (verkratzt, abgerieben) werden.

Sehr kleine Strukturen über mehrere Ebenen lassen sich nur mittels eines mehrstufigen Prozesses oder mittels Fotolithografie herstellen. Für die Fotolithographie wird die Isolationskeramik teilweise oder vollständig mit einem Photoresist beschichtet. Dieser wird belichtet, ggf. entwickelt. Die verbleibende Photoresistschicht wird entfernt und dient als Maskierung, welche nach dem Beschichten wieder vom Bauteil gestrippt werden kann.

Der Erfindung liegt die Aufgabe zugrunde, ein verbessertes Verfahren der gattungsgemäßen Art anzugeben, welches insbesondere ein Durchführungsbauteil mit vorteilhaften, klar definierten elektrischen Eigenschaften liefert, insbesondere derart, dass die Isolationseigenschaften der Durchführung gezielt eingestellt werden können. Das bevorzugtermaßen auch besonders kompakt gestaltet werden kann und/oder die Aufnahme einer großen Anzahl von Anschlusselementen mit hoher Funktionssicherheit erlauben soll.

Diese Aufgabe wird durch ein Verfahren mit den Merkmalen des Anspruchs 1 gelöst. Zweckmäßige Fortbildungen des Verfahrens sind Gegenstand der abhängigen Verfahrensansprüche. Es wird weiterhin ein neuartiges Durchführungsbauteil mit den Merkmalen des Anspruchs 12 vorgeschlagen. Zweckmäßige Fortbildungen des vorgeschlagenen Durchführungsbauteils sind Gegenstand der abhängigen Produktansprüche.

Die Erfindung schließt den Gedanken eines Abgehens sowohl von den bekannten Verfahren mit Vorab-Strukturierung einer Metallbeschichtung bereits bei deren Aufbringen als auch von den Verfahren mit mechanischer Strukturierung einer vorab großflächig aufgebrachten Metallbeschichtung ein. Sie schließt weiter den Gedanken ein, an die Stelle jener Verfahren ein solches zu setzen, bei dem eine Strukturierung bzw. Modifizierung der vorab großflächig aufgebrachten Metallbeschichtung mittels eines Laserbearbeitungsverfahrens erfolgt. Insgesamt wird erfindungsgemäß vorgeschlagen, dass die Strukturierung durch schichtweises, mindestens partielles Abtragen der Metallbeschichtung in mehreren Teilschritten mittels eines Bearbeitungslasers ausgeführt wird.

Mit dem erfindungsgemäßen Verfahren lässt sich wenigstens einer der nachfolgend genannten Vorteile, in bevorzugten Ausführungen eine Kombination mehrerer Vorteile, erzielen:
- Der Hochtemperaturlötprozess unterliegt weniger Schwankungen. Die Ausbeute des Prozesses wird durch eine bessere Qualität der Vorprodukte erhöht.
   Die Eigenschaften der Durchführungen, insbesondere die elektrischen, lassen sich bereits am Halbzeug gezielt einstellen oder manipulieren. Eine gezielt nachbearbeitete Oberfläche kann die Variation der Prozessergebnisse aus der Beschichtung reduzieren.
- Es können Werkstoffe mit abweichenden Wärmeausdehnungskoeffizienten verarbeitet werden. Die Materialien mussten nicht mehr notwendigerweise aufeinander abgestimmt werden, damit die thermische Belastung nicht zu Rissen und Verwerfungen führt.
- Durch Dehnungsfugen und konstruktive Gestaltung kann der Weg des Hartlotes während des Fügens unterbrochen, kontrolliert und manipuliert werden.
- Große Metallisierungsflächen können durch eine Vielzahl kleinere Geometrien mit gleicher Fläche ersetzt werden.
- Es müssen keine Abdeckkappen bzw. Maskierungen mehr hergestellt und verwaltet werden.

In einer Ausführung des Verfahrens werden mindestens bei einigen Teilschritten unterschiedliche, schritt-spezifische Betriebsparameter des Bearbeitungslasers eingestellt. Dies gilt insbesondere dann, wenn die Metallbeschichtung Teilschichten unterschiedlicher Zusammensetzung und/oder unterschiedlicher Dicke und/oder unterschiedlicher Lage relativ zu einer Bezugsebene (etwa der Ebene eines Werkstücktisches) aufweist oder wenn in den Teilschritten unterschiedliche Aspekte der auszuführenden Strukturierung realisiert werden.

In einer aus derzeitiger Sicht bevorzugten Verfahrensführung werden Konturen des Durchführungs-Grundkörpers und/oder der Metallbeschichtung mit einem Scanner abgetastet, und der Bearbeitungslaser wird mittels des Scanners positionsgesteuert. Derartige Werkzeug-Steuerungen mittels Werkstückabtastung durch einen Scanner (und eines Vergleiches mit gespeicherten Werkstückgeometrien und Bearbeitungsparametern) sind an sich bekannt und bedürfen daher hier keiner weiteren Erläuterung für den Fachmann.

In einer Ausgestaltung der erwähnten Ausführung wird die Situation gemeistert, dass in einem Randbereich einer partiellen Metallbeschichtung oder in einem Stufen-Randbereich einer Metallbeschichtung mit abgestufter Dicke eine geringere Dicke vorliegt oder die Metallbeschichtung lokal unterbrochen ist. Dann wird der Bearbeitungslaser derart gesteuert, dass der Randbereich oder Stufen-Randbereich mit geringerer Dicke oder mit Unterbrechungen modifiziert wird. Dies geschieht derart, dass der Rand oder Stufen-Rand eine vordefinierte Kontur und/oder im Wesentlichen die gleiche Dicke wie der jeweilige Mittenbereich der Metallbeschichtung oder eine mit vordefiniertem Verlauf abnehmende Dicke erhält.

In einer weiteren Ausgestaltung wird im Falle eines Abtragens der Metallbeschichtung in der gesamten Dicke auch eine Oberflächenschicht des Durchführungs-Grundkörpers mit abgetragen. Insbesondere wird hierzu die Fokusebene des Bearbeitungslasers mindestens in einem letzten Abtragungsschritt unterhalb der Grenzfläche zwischen der Metallbeschichtung und Durchführungs-Grundkörper eingestellt.

In einer anderen Ausführungsform umfasst die Strukturierung eine oberflächliche lokale oder bereichsweise Dickenverringerung der Metallbeschichtung. Die Metallbeschichtung wird also bei dieser Ausgestaltung, die mit der vorgenannten Ausgestaltung kombiniert sein kann, in den entsprechenden Bereichen nicht bis auf die Isolationskeramik abgetragen, sondern lediglich abgedünnt bzw. - im Falle einer vorher ungleichmäßig dicken Beschichtung - egalisiert.

In einer anderen Ausführung umfasst die Strukturierung eine lokale oder bereichsweise Dickenvergrößerung der Metallbeschichtung. Diese wird durch ein abschnitts- und/oder schichtweises Aufschmelzen der Metallbeschichtung und Verschieben der aufgeschmolzenen Abschnitte bei der Abtastung des Durchführungs-Bauteils mit dem Bearbeitungslaser bewirkt. In einer praktisch bedeutsamen Ausführung wird hierbei mindestens ein Randbereich der Metallbeschichtung mit einer vergrößerten Dicke versehen. Es kann auch eine Egalisierung unebener Schichtoberflächen bewirkt werden.

In einer weiteren Ausführung werden zur Strukturierung einer Metallbeschichtung, die auf unterschiedlichen Höhenstufen des Durchführungs-Grundkörpers vorgesehen ist, während des Strukturierungsvorganges lokal oder bereichsweise verschiedene Fokusebenen eingestellt.

Gemäß einem relativ unabhängigen Aspekt der Erfindung weist die Metallbeschichtung eines neuartigen Durchführungsbauteils eine nachträglich durch lokales Aufschmelzen und Verdampfen von Metall der Metallbeschichtung eingeprägte Struktur auf. Ein solches Durchführungsbauteil kann vorteilhaft mit dem vorstehend erläuterten Verfahren gebildet werden, seine Bereitstellung ist aber nicht notwendigerweise an die Anwendung dieses Verfahrens gebunden.

In einer Ausführung weist die eingeprägte Struktur auch Bereiche auf, in denen Material des Durchführungs-Grundkörpers oberflächlich abgetragen ist.

In weiteren Ausführungen ist in Randbereichen der strukturierten Metallbeschichtung deren Dicke gegenüber Mittenbereichen vergrößert oder mit vordefiniertem Verlaufsgradienten abnehmend und/oder mit einer vordefinierten Kontur versehen.

In weiteren Ausführungen des vorgeschlagenen Durchführungsbauteils ist innerhalb der Metallbeschichtung mindestens eine Dehnungsfuge zum Ausgleich unterschiedlicher thermischer Ausdehnungskoeffizienten des Durchführungs-Grundkörpers und der Metallbeschichtung eingearbeitet. Diese Ausführung ermöglicht in vorteilhafter Weise eine Bauteilkonstruktion, bei der Funktionsstörungen der Durchführung in Folge häufiger thermischer Wechselbelastung weitgehend ausgeschlossen sind.

Vorteile und Zweckmäßigkeiten der Erfindung ergeben sich im Übrigen aus der nachfolgenden Beschreibung eines Ausführungsbeispiels anhand der Figuren. Von diesen zeigen
- Fig. 1: eine schematische, teilweise geschnittene Darstellung eines implantierbaren elektromedizinischen Geräts;
- Fig. 2: ein Ausführungsbeispiel eines erfindungsgemäß hergestellten Durchführungsbauteils, als längs geschnittene perspektivische Ansicht,
- Fig. 3: zwei schematische Darstellungen von beschichteten Isolationskeramiken einer elektrischen Durchführung,
- Fig. 4: eine perspektivische Darstellung einer weiteren beschichteten Isolationskeramik,
- Fig. 5: eine perspektivische Darstellung einer weiteren beschichteten Isolationskeramik,
- Fig. 6: eine schematische Darstellung einer weiteren beschichteten Isolationskeramik,
- Fig. 7: eine weitere perspektivische Darstellung einer beschichteten Isolationskeramik, und
- Fig. 8: Darstellungen und ein Tiefenprofil zur Verdeutlichung der geometrischen Charakteristika eines Ausführungsbeispiels einer erfindungsgemäß beschichteten Isolationskeramik.

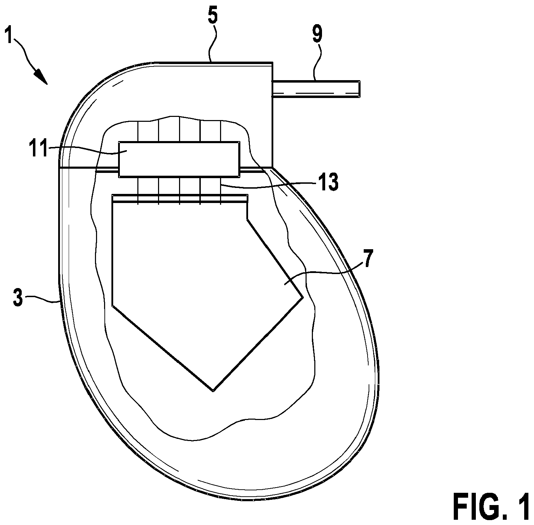

Fig. 1 zeigt einen Herzschrittmacher 1 mit einem Schrittmachergehäuse 3 und einem Kopfteil (Header) 5, in dessen Innerem neben anderen elektronischen Komponenten eine Leiterplatte (PCB) 7 angeordnet und mit dessen im Header angeordneten (nicht gezeigten) Leitungsanschluss eine Elektrodenleitung 9 verbunden ist. Eine zwischen Gerätegehäuse 3 und Header 5 vorgesehene Durchführung 11 umfasst eine Mehrzahl von Anschlussstiften 13. Die Anschlussstifte sind an einem Ende durch eine entsprechende Bohrung in der Leiterplatte gesteckt und mit dieser verlötet.

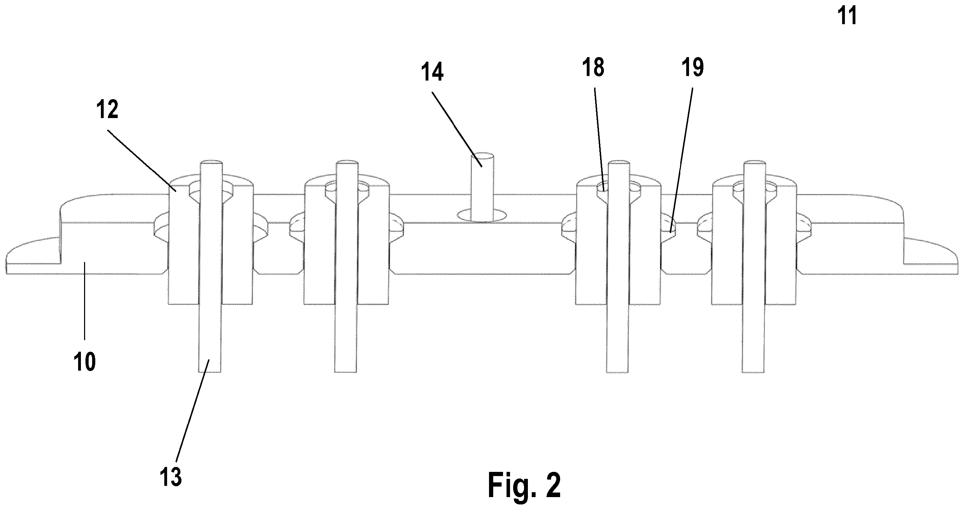

Fig. 2 zeigt eine elektrische Durchführung 11 bestehend aus einem Flansch 10, Isolationskeramiken 12, Signalstiften 13 und einem Massestift 14. Die Signalstifte 13 sind jeweils mit einem Hartlot 18 in die Isolationskeramik 12 gefügt, ebenso wie die Isolationskeramik 12 mit einem Hartlot 19 in den Flansch gefügt ist. Üblicherweise wird auch der Massestift 14 mit einem Hartlot an den Flansch kontaktiert.

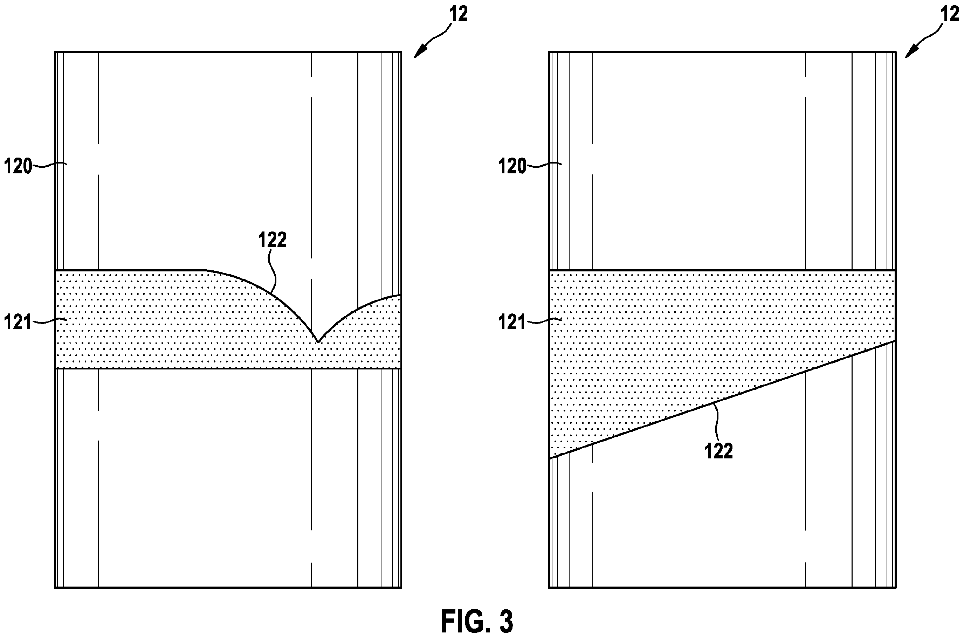

Fig. 3 ist die schematische Darstellung zweier beschichteten Isolationskeramiken 12 einer elektrischen Durchführung. Die Isolationskeramiken bestehen aus keramischen Substrat 120 und einer Metallisierung 121. Die Metallisierung 121 kann mittels eines PVD-Prozesses aufgebracht sein. Prozessbedingt können sich im PVD-Prozess Beschichtungsfehler 122 bilden. Diese führen zu einer veränderten Beschichtungsfläche und damit zu ungleichmäßigen Ergebnissen während des Hartlötprozesses der medizinischen Durchführung.

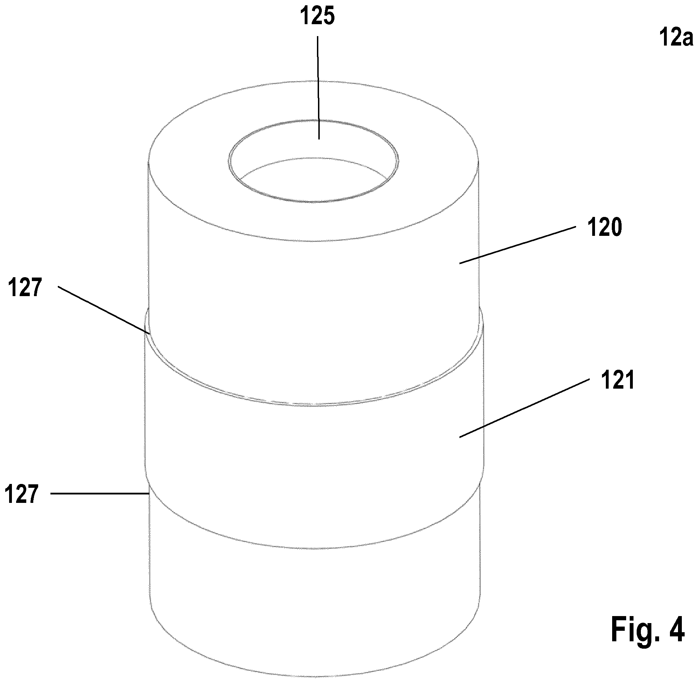

Fig. 4 ist die schematische Darstellung einer beschichteten Isolationskeramik 12a. Auf das keramische Substrat 120 wurde eine Metallisierung 121 aufgebracht. Um eine scharfe Begrenzung der Metallisierung 121 zu erreichen, wurden jeweils an den Rändern der Metallisierung 121 umlaufend eine Entschichtung 127 durchgeführt. Durch die Entschichtung 127 wird die Fläche der Beschichtung verkleinert, aber ein kontrastreicher, scharf abgegrenzter Übergang zwischen Keramik und Beschichtung erreicht. Die Höhe des Absatzes ist sowohl durch die Anzahl und Dichte der Laserpulse auf der Metallisierung 121 als auch durch die Anzahl und Dichte der Laserpulse auf das keramische Substrat 120 einstellbar.

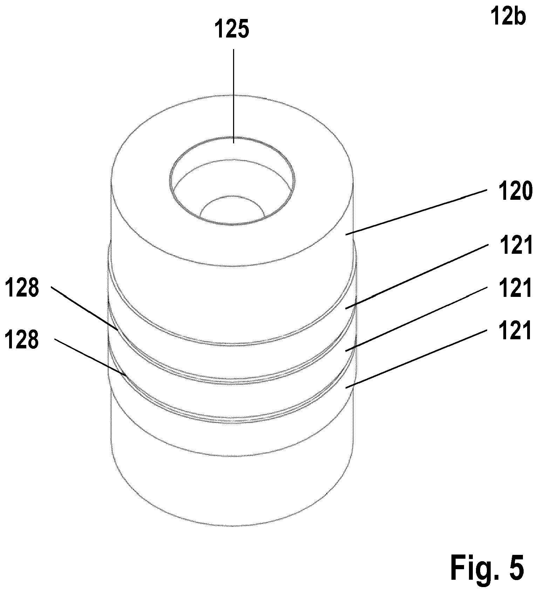

Fig. 5 ist die schematische Darstellung einer beschichteten Isolationskeramik 12b. Auf das keramische Substrat 120 wurde eine Metallisierung 121 aufgebracht. Um die Gefahr von Rissen in der Metallisierung 121 auf Grund von Spannungen zu vermeiden, wird die Metallisierung 121 durch Fugen 128 unterbrochen. Die Eindringtiefe in die Metallisierung bzw. in das keramische Substrat ist durch die Anzahl und Dichte der Laserpulse einstellbar.

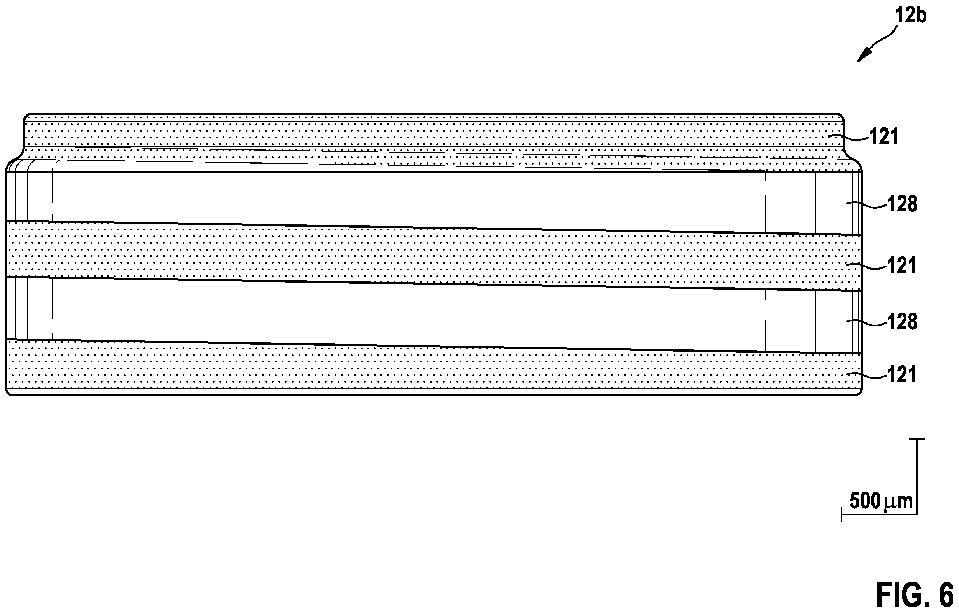

Fig. 6 ist die schematische Darstellung einer beschichteten Isolationskeramik. Die Metallisierung 121 wird durch Fugen 128 unterbrochen.

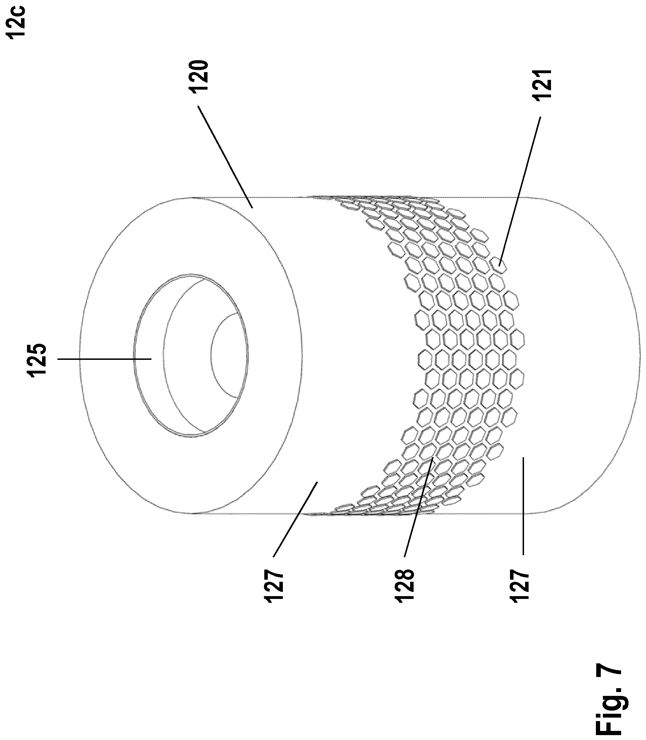

Fig. 7 ist die schematische Darstellung einer beschichteten Isolationskeramik 12. Auf der Mantelseite des keramischen Substrates wird eine Metallisierung aufgebracht. Durch Entschichtung 127 der nicht benötigten Bereiche wird das Keramiksubstrat freigelegt. Die verbliebene Beschichtung wird durch mehrere Fugen 128 freigelegt und strukturiert. Die verbleibende Metallisierung 121 setzt sich aus einer Vielzahl einzelner Flächen zusammen.

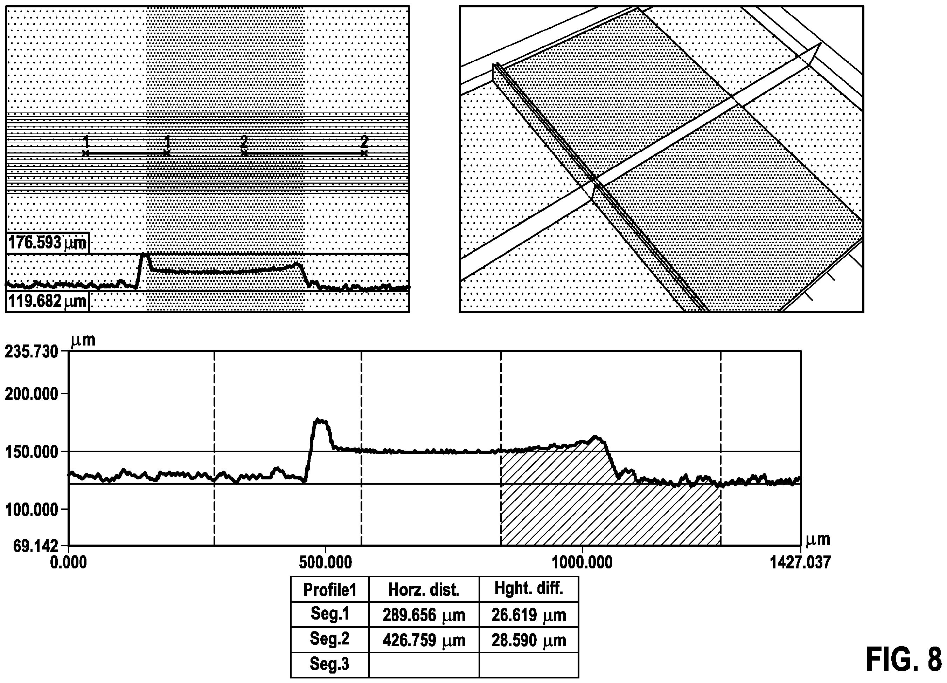

Fig. 8 zeigt das Tiefenprofil auf einer Isolationskeramik. Vermessen wurde der Höhenunterschied zwischen der Metallisierung 121 und zwei Entschichtungsbereichen 127. Die Metallisierungsstärke auf dem Substrat beträgt 12 µm ± 3 µm. Zur vollständigen Entschichtung muss schichtweise etwa 15 µm ± 3 µm in die Keramik gelasert werden. Die Rauheit der des einschichten Substrates ist höher als das der Rohkeramik.

Deutlich zu erkennen ist die Überhöhung auf der linken Seite der Metallisierung 121. Sie wurde durch das wiederholte Entschichten des linken Bereiches von links nach rechts erzeugt.

Ausführungen des vorgeschlagenen Verfahrens können insbesondere die folgenden Aspekte aufweisen:
Die Isolationskeramik wird teilweise oder ganz mit mindestens einer Metallisierung oder einer Legierung beschichtet. Vorzugsweise werden biokompatible Metalle (z. B. Nb, Ti, Wo) eingesetzt. Vorteilhaft sind Metalle deren Wärmeausdehnungskoeffizient nahe dem des Keramiksubstrates liegen.

Vorteilhaft kann es sein, die Metallisierung aus mehreren Schichten aufzubauen Die typische Schichtdicke der Metallisierung beträgt einige Mikrometer.

Nach dem Beschichten wird die Isolationskeramik mittels eines Laserstrahls schichtweise abgetragen. Hierzu rastert ein Laserstrahl die beschichtete Keramik ab, dabei wird die Metallisierung teilweise oder ganz von der Keramik entfernt. Hierzu eignet sich ein konventioneller Beschriftungslaser im Pulsbetrieb (z. B. ein Faserlaser). Die Ausrichtung des Lasers auf dem Bauteil erfolgt mittels eines Scanners. Dieser positioniert den Laser und lenkt den Fokus in mehreren Achsen über das Bauteil.

Mit dem Laser kann die die Beschichtung teilweise oder ganz abgetragen werden. Ferner kann die Beschichtung teilweise oder flächig mikrostrukturiert werden. Vorteilhaft ist es, in der Metallisierung Bereiche mit unterschiedlicher Dicke zu erzeugen, um die Lösungsgeschwindigkeit im Hartlot besser steuern zu können.

Vorteilhaft kann es auch sein, in Beschichtungsmaterialen mit einem vom Substrat abweichenden Wärmeausdehnungskoeffizienten definierte Trennfugen in die Beschichtung einzuarbeiten, die ein unkontrolliertes Abreißen oder Ablösen der Schicht während des Hartlötens verhindern.

Von Vorteil kann es sein, die typischen Übergangszonen des PVD-Maskenprozesses mit langsam auslaufender Beschichtungsstärke und/oder unregelmäßigen Kanten nachzubearbeiten, so dass definierte Materialübergänge entstehen.

Von Vorteil ist es weiterhin, die Metallisierung auf der Isolationskeramik teilweise, abschnittsweise oder vollflächig zu strukturieren, um die elektrischen Eigenschaften der Durchführung zu verbessern oder das Ergebnis des anschließenden Hartlötprozess positiv zu beeinflussen.

Die Wiederholgenauigkeit von preiswerten Laserscannersystemen liegt typischerweise bei wenigen 0,001 mm und die Tiefenschärfe der Laserscanner beträgt einige Millimeter, so dass die Entschichtung bzw. Nacharbeit deutlich bessere Ergebnisse liefert als ein konventioneller Maskierungsprozess.

Da beim Abtragen der Beschichtung mittels eines Lasers Verschmutzungen auf das umliegende Material entstehen, kann es von Vorteil sein, einige Fokusebenen (typischerweise 1x-10x) tiefer ins das Substrat abzutragen, so dass die Metallisierung und deren Abdampfungen bzw. Spritzer restlos entfernt werden

### Homogenisierung

Durch gezielte Ablation der Schicht mit einem Faser- oder Ultrakurzpulslaser (UKP) kann die Schichtdicke der Beschichtung verringert oder lokal überhöht werden. Durch die gezielte Überarbeitung der Schichtdicke können kleine Defekte in der Beschichtung ausgeglichen und Unregelmäßigkeiten in der Beschichtung abgetragen werden. Durch das Abrastern der gesamten Beschichtung in einer definierten Fokusebene kann eine gleichmäßige homogene Näpfchen- oder Linienstruktur mit einer einstellbaren definierten Restunebenheit auf dem Bauteil erzeugt werden.

### Kantenkontrast

Prozessbedingt kann es beim Beschichten mittels PVD-Verfahren mittels Maskenprozess zu einem unregelmäßigen Auslaufen der Beschichtung kommen. Der Übergangsbereich ist je nach Qualität der Masken und Beschichtungsstärke beträgt typischerweise einige µm und kann versetzt zu den Außenkanten der Isolationskeramik liegen.

Durch gezieltes Nacharbeiten mit dem Laser, lässt sich der Gradient zwischen den Bereichen vergrößern und die Übergangszone eliminieren. Der an die Beschichtung grenzende Bereich wird mit dem Laser abgerastert und die ggf. vorhandenen Verunreinigen oder Rückstände werden schichtweise abgetragen. Um den Kontrast noch weiter zu steigern, ist es von Vorteil, neben dem an der Beschichtung angrenzenden Bereich auch im Beschichtungsbereich das Grundmaterial freizulegen und einige µm der Keramik zu sublimieren.

Der verbleibende Beschichtungsbereich grenzt sich so sehr deutlich und kontrastreich von der Keramik ab. Neben dem optischen Unterschied auf Grund des Materials, kann der Höhenunterschied zwischen Beschichtung und Isolationskeramik so verstärkt werden. Der Beschichtungsbereich kann manuell oder während einer automatischen optischen Inspektion zuverlässiger erkannt werden.

Vorteilhaft kann es sein, die Kanten zwischen Beschichtung und Isolationskeramik nicht als gerade Linie, sondern mit einer vordefinierten regelmäßigen Kontur auszuführen.

Vorteilhaft kann es sein, eine Übergangszone mit definiertem Gradienten zu erzeugen. Hierzu wird die Schichtdicke im Bereich einer Aus- oder Einlaufzone gezielt verringert, so dass ein definierter weicher Kantenkontrast entsteht.

### Schichtüberhöhung

Durch das Aufschmelzen und Räumen von einer größeren Fläche mittels eines gepulsten Lasers kann die Schichtdicke lokal auch überhöht werden.

Hierzu wird die beschichtete Isolationskeramik mit dem Laser abgerastert und die Beschichtung schichtweise aufgeschmolzen. Durch geschickte Wahl der Laserparameter, kann ein Teil der aufgeschmolzenen Beschichtung in die Vorschubrichtung des Strahles verschoben werden, wo sie erkaltet.

Vorteilhaft ist es, die Beschichtung am Rand zu überhöhen, um einen deutlichen Kontrast für optische Kontrollen zu erzielen. Vorteilhaft ist es ggf. auch, eine lokale Überhöhung zu erzeugen, um die Abtragung durch das Anlösen der Beschichtung im Hartlot zu kompensieren.

### Dehnungsfugen

Die Isolationskeramik und die Beschichtung bestehen aus verschiedenen Werkstoffen und haben verschiedene Wärmeausdehnungskoeffizienten. Für große oder zusammenhängende Flächen ist daher technisch notwendig, Werkstoffe mit zumindest ähnlichen Ausdehnungskoeffizienten zu wählen, um die Gefahr des unkontrollierten Schichtablösen bzw. Schichteinrisses während bzw. nach dem Fügen zu verringern bzw. zu minimieren. Durch das Einfügen einer Dehnungsfuge (kontrolliertes Trennen von Beschichtung und Isolationskeramik) an definierten Bereichen kann die Gefahr des unkontrollierten Schichtablösens, Schichtabplatzen oder Schichteinrisses während des Lötens eingeschränkt werden. Da die Dehnfugen verhindern, dass ein Wärmeriss zwischen Beschichtung und Isolationskeramik aufgrund von unterschiedlichen Wärmeausdehnungskoeffizienten sich unkontrolliert im Material fortpflanzt, wird es möglich, auch Materialpaarungen mit unterschiedlichen Wärmeausdehnungskoeffizienten einzusetzen.

Durch die Dehnungsfuge werden Materialverwerfungen auf der Beschichtung, insbesondere bei Mehrmetallschichtsystemen vermieden.

### Zusammengesetzte bzw. segmentierte Flächen

Durch das Strukturieren der beschichteten Fläche kann die Beschichtung der Mantelfläche in kleine Flächen aufgeteilt werden. Anstatt einer großen zusammenhängenden Beschichtung kann die Beschichtung aus vielen kleinen Einzelflächen zusammengesetzt werden. Die Gefahr von Rissen in der Beschichtung aufgrund der unterschiedlichen Wärmeausdehnung kann so minimiert werden.

Das Hartlot (z. B. Gold), welches später in den Spalt zwischen Keramik und Flansch fließt, fügt die Komponenten weiterhin zuverlässig zu einem hermetisch dichten Bauteil. Durch die Oberflächenspannung des flüssigen Lotes werden die Strukturierungen überbrückt.

Vorteilhaft ist es ggf., wenn die Richtung der Strukturierung senkrecht zur Lotflussrichtung liegt, um ein Durchfließen durch den Lötspalt zu verhindern. Vorteilhaft kann es aber auch sein, wenn die Richtung der Strukturierung in Lotflussrichtung liegt, um ein Einfließen in den Lötspalt zu begünstigen.

Vorteilhaft kann es sein, durch eine zusammengesetzte bzw. segmentierte Fläche die effektive Strecke durch den Lötspalt zu verlängern.

### Trimmung

Um die Streuung der Produkteigenschaften möglichst gering zu halten, ist es notwendig die Isolationskeramik mit möglichst konstanten elektrischen Eigenschaften und Fertigungstoleranzen zu fertigen. Da der Beschichtungsbereich direkten Einfluss auf die elektrischen Eigenschaften und auf die Prozessstabilität des Hartlötens erzeugt, ist es wichtig diesen möglichst reproduzierbar einstellen zu können.

Im Übrigen ist die Ausführung der Erfindung auch in einer Vielzahl von Abwandlungen der hier gezeigten Beispiele und weiter oben hervorgehobenen Aspekte der Erfindung möglich.

## Patentansprüche

1. Verfahren zur Herstellung eines Durchführungsbauteils (11) eines medizinelektronischen Gerätes, wobei ein Durchführungs-Grundkörper aus Keramik bereitgestellt und großflächig mit einer Metallbeschichtung versehen und die Metallbeschichtung anschließend strukturiert wird, wobei die Strukturierung durch schichtweises, mindestens partielles Abtragen der Metallbeschichtung in mehreren Teilschritten mittels eines Bearbeitungslasers ausgeführt wird,
**dadurch gekennzeichnet, dass**
während der Strukturierung der Metallbeschichtung innerhalb der Metallbeschichtung mindestens eine Dehnungsfuge (128) zum Ausgleich unterschiedlicher thermischer Ausdehnungskoeffizienten des Durchführungs-Grundkörpers und der Metallbeschichtung eingearbeitet wird,
wobei das Durchführungsbauteil (11) im Anschluss über die Strukturierte Metallbeschichtung mittels eines Hartlötprozesses mit einem Flansch (10) und/oder Signalstift (13) gefügt wird.

2. Verfahren nach Anspruch 1, wobei mindestens bei einem Teil der Teilschritte unterschiedliche, schritt-spezifische Betriebsparameter des Bearbeitungslasers eingestellt werden.

3. Verfahren nach Anspruch 1 oder 2, wobei Konturen des Durchführungs-Grundkörpers und/oder der Metallbeschichtung mit einem Scanner abgetastet werden und der Bearbeitungslaser mittels des Scanners positionsgesteuert wird.

4. Verfahren nach einem der vorangehenden Ansprüche, wobei die Metallbeschichtung bereichsweise eine unterschiedliche Dicke aufweist und Betriebsparameter des Bearbeitungslasers beim Abtasten der Metallbeschichtung in Abhängigkeit von der lokalen Dicke eingestellt werden.

5. Verfahren nach Anspruch 4, wobei in einem Randbereich einer partiellen Metallbeschichtung oder in einem Stufen-Randbereich einer Metallbeschichtung mit abgestufter Dicke eine geringere Dicke vorliegt oder die Metallbeschichtung lokal unterbrochen ist und der Bearbeitungslaser derart gesteuert wird, dass der Randbereich oder Stufen-Randbereich mit geringerer Dicke oder mit Unterbrechungen derart modifiziert wird, dass der Rand oder Stufen-Rand eine vordefinierte Kontur und/oder im Wesentlichen die gleiche Dicke wie der jeweilige Mittenbereich der Metallbeschichtung oder eine mit vordefiniertem Verlauf abnehmende Dicke erhält.

6. Verfahren nach einem der vorangehenden Ansprüche, wobei im Falle eines Abtragens der Metallbeschichtung in ihrer gesamten Dicke auch eine Oberflächenschicht des Durchführungs-Grundkörpers mit abgetragen wird.

7. Verfahren nach Anspruch 6, wobei die Fokusebene des Bearbeitungslasers mindestens in einem letzten Abtragungsschritt unterhalb der Grenzfläche zwischen der Metallbeschichtung und Durchführungs-Grundkörper eingestellt wird.

8. Verfahren nach einem der Ansprüche 1 bis 5, wobei die Strukturierung eine Oberflächliche lokale oder bereichsweise Dickenverringerung der Metallbeschichtung umfasst.

9. Verfahren nach einem der Ansprüche 1 bis 5, wobei die Strukturierung eine lokale oder bereichsweise Dickenvergrößerung der Metallbeschichtung umfasst, welche durch ein abschnitts- und/oder schichtweises Aufschmelzen der Metallbeschichtung und Verschieben der aufgeschmolzenen Abschnitte bei der Abtastung des Durchführungs-Bauteils mit dem Bearbeitungslaser bewirkt wird.

10. Verfahren nach Anspruch 9, wobei mindestens ein Randbereich der Metallbeschichtung mit einer vergrößerten Dicke versehen wird.

11. Verfahren nach einem der vorangehenden Ansprüche, wobei zur Strukturierung einer Metallbeschichtung, die auf unterschiedlichen Höhenstufen des Durchführungs-Grundkörpers vorgesehen ist, während des Strukturierungsvorganges lokal oder bereichsweise verschiedene Fokusebenen eingestellt werden.

12. Durchführungsbauteil (11) eines medizinelektronischen Gerätes, welches einen Durchführungs-Grundkörper aus Keramik aufweist, der großflächig mit einer strukturierten Metallbeschichtung versehen ist, hergestellt nach einem der vorangehenden Ansprüche, wobei die Metallbeschichtung eine nachträglich durch lokales Aufschmelzen und/oder Verdampfen von Metall der Metallbeschichtung eingeprägte Struktur aufweist,
**dadurch gekennzeichnet, dass**
das innerhalb der Metallbeschichtung mindestens eine Dehnungsfuge (128) zum Ausgleich unterschiedlicher thermischer Ausdehnungskoeffizienten des Durchführungs-Grundkörpers und der Metallbeschichtung eingearbeitet ist,
wobei das Durchführungsbauteil (11) mittels eines Hartlots über die Strukturierte Metallbeschichtung mit einem Flansch (10) und/oder Signalstift (13) gefügt ist.

13. Durchführungsbauteil nach Anspruch 12, wobei die eingeprägte Struktur auch Bereiche aufweist, in denen Material des Durchführungs-Grundkörpers oberflächlich abgetragen ist.

14. Durchführungsbauteil nach Anspruch 12 oder 13, wobei in Randbereichen der strukturierten Metallbeschichtung deren Dicke gegenüber Mittenbereichen vergrößert oder mit vordefiniertem Verlaufsgradienten abnehmend und/oder mit einer vordefinierten Kontur versehen ist.

## Claims

1. A method for producing a feedthrough component (11) of a medical electronic device, wherein a feedthrough main body made of ceramic is produced and is provided over a large area with a metal coating, and the metal coating is then structured, wherein the structuring is performed by at least partially removing the metal coating in layers in a number of sub-steps by means of a processing laser,
**characterised in that**
during the structuring of the metal coating, at least one expansion joint (128) is formed within the metal coating in order to compensate for different coefficients of thermal expansion of the feedthrough main body and the metal coating,
wherein the feedthrough component (11) is then joined to a flange (10) and/or signal pin (13) via the structured metal coating by means of a brazing process.

2. The method according to claim 1, wherein different, step-specific operating parameters of the processing laser are adjusted at least in some of the sub-steps.

3. The method according to claim 1 or 2, wherein contours of the feedthrough main body and/or the metal coating are scanned by a scanner and the position of the processing laser is controlled by means of the scanner.

4. The method according to any one of the preceding claims, wherein the metal coating has a different thickness in regions, and operating parameters of the processing laser when scanning the metal coating are adjusted depending on the local thickness.

5. The method according to claim 4, wherein a smaller thickness is provided or the metal coating is locally interrupted in an edge region of a partial metal coating or in a step edge region of a metal coating with a stepped thickness, and the processing laser is controlled in such a way that the edge region or step edge region with smaller thickness or with interruptions is modified in such a way that the edge or step edge obtains a predefined contour and/or substantially the same thickness as the corresponding middle region of the metal coating or obtains a thickness that decreases with a predefined course.

6. The method according to any one of the preceding claims, wherein in the case of a removal of the metal coating over its entire thickness, a surface layer of the feedthrough main body is also removed at the same time.

7. The method according to claim 6, wherein the focal plane of the processing laser, at least in a last removal step, is set below the boundary between the metal coating and feedthrough main body.

8. The method according to any one of claims 1 to 5, wherein the structuring comprises a local or regional thickness reduction of the metal coating at the surface.

9. The method according to any one of claims 1 to 5, wherein the structuring comprises a local or regional increase in thickness of the metal coating, which is caused by melting the metal coating in portions and/or in layers and displacing the melted portions during the scanning of the feedthrough component with the processing laser.

10. The method according to claim 9, wherein at least one edge region of the metal coating is provided with an increased thickness.

11. The method according to any one of the preceding claims, wherein different focal planes are set locally or regionally during the structuring process in order to structure a metal coating provided at different heights of the feedthrough main body.

12. A feedthrough component (11) of a medical electronic device, which has a feedthrough main body made of ceramic, which is provided over a large area with a structured metal coating, produced in accordance with one of the preceding claims, wherein the metal coating has a structure impressed subsequently by local melting and/or evaporation of metal of the metal coating,
**characterised in that**
at least one expansion joint (128) is formed within the metal coating in order to compensate for different coefficients of thermal expansion of the feedthrough main body and the metal coating,
wherein the feedthrough component (11) is then joined to a flange (10) and/or signal pin (13) via the structured metal coating by means of a brazing process.

13. The feedthrough component according to claim 12, wherein the impressed structure also has regions in which material of the feedthrough main body is removed at the surface.

14. The feedthrough component according to claim 12 or 13, wherein the thickness of the structured metal coating in edge regions is increased compared to middle regions or decreases with a predefined gradient of progression and/or is provided with a predefined contour.

## Revendications

1. Procédé de fabrication d'un élément constitutif de passage (11) d'un appareil médical électronique, dans lequel un corps de base de passage est préparé à base de céramique et est muni d'un revêtement métallique sur toute la surface et ensuite le revêtement métallique est structuré, où la structuration est effectuée par un retrait du revêtement métallique par couches au moins partiel en plusieurs étapes partielles au moyen d'un laser de façonnage,
**caractérisé en ce que,**
pendant la structuration du revêtement métallique, au moins un joint de dilatation (128) est incorporé à l'intérieur du revêtement métallique pour la compensation de coefficients de dilatation thermique variables du corps de base de passage et du revêtement métallique,
où l'élément constitutif de passage (11) est joint au niveau du raccord avec une bride (10) et/ou une tige de signalisation (13) par l'intermédiaire du revêtement métallique structuré au moyen d'un procédé de brasage.

2. Procédé selon la revendication 1, dans lequel, lors d'au moins une partie des étapes partielles, on règle des paramètres de fonctionnement divers, spécifiques à l'étape, du laser de façonnage.

3. Procédé selon la revendication 1 ou 2, dans lequel des contours du corps de base de passage et/ou du revêtement métallique sont relevés par balayage avec un scanner et le laser de façonnage est commandé en ce qui concerne les positions au moyen du scanner.

4. Procédé selon l'une des revendications précédentes, dans lequel le revêtement métallique présente par endroits une épaisseur variable et des paramètres de fonctionnement du laser de façonnage sont réglés lors du relevé par balayage du revêtement métallique en fonction de l'épaisseur locale.

5. Procédé selon la revendication 4, dans lequel il y a une épaisseur plus faible dans une zone de bordure d'un revêtement métallique ou dans une région de bordure en dénivelé d'un revêtement métallique avec une épaisseur décroissante par paliers ou le revêtement métallique est interrompu localement et le laser de façonnage est commandé de telle manière que la région de bordure ou la région de bordure en dénivelé est modifiée avec une épaisseur moindre ou avec des interruptions, de sorte que la bordure ou la bordure en dénivelé a un contour prédéfini, et/ou a dans l'ensemble la même épaisseur que la région centrale respective du revêtement métallique, ou a une épaisseur diminuant avec un profil prédéfini.

6. Procédé selon l'une des revendications précédentes, dans lequel, dans le cas d'un retrait du revêtement métallique sur son épaisseur entière, une couche de surface du corps de base de passage est retirée conjointement.

7. Procédé selon la revendication 6, dans lequel le plan focal du laser de façonnage est réglé au moins dans une dernière étape de retrait en-dessous de la surface limite entre le revêtement métallique et le corps de base de passage.

8. Procédé selon l'une des revendications 1 à 5, dans lequel la structuration comprend une diminution d'épaisseur du revêtement métallique superficielle locale ou par endroits.

9. Procédé selon l'une des revendications 1 à 5, dans lequel la structuration comprend un accroissement d'épaisseur du revêtement métallique local ou par endroits, lequel est créé par une fusion du revêtement métallique par endroits et/ou par couches et par un déplacement des segments fondus lors du relevé par balayage de l'élément constitutif de passage avec le laser de façonnage.

10. Procédé selon la revendication 9, dans lequel au moins une région de bordure du revêtement métallique est munie d'une épaisseur plus grande.

11. Procédé selon l'une des revendications précédentes, dans lequel, pour la structuration d'un revêtement métallique qui est prévue pour différents dénivelés du corps de base de passage, des plans focaux différents sont réglés localement ou par endroits pendant le processus de structuration.

12. Elément constitutif de passage (11) d'un appareil médical électronique, lequel présente un corps de base de passage à base de céramique qui est muni sur toute la surface d'un revêtement métallique structuré fabriqué selon l'une des revendications précédentes, où le revêtement métallique présente ensuite une structure apportée par une fusion locale et/ou une évaporation de métal du revêtement métallique,
**caractérisé en ce**
**qu'**au moins un joint de dilatation (128) est incorporé à l'intérieur du revêtement métallique pour la compensation de coefficients de dilatation thermique variables du corps de base de passage et du revêtement métallique,
où l'élément constitutif de passage (11) est joint avec une bride (10) et/ou une tige de signalisation (13) au moyen d'un brasage par l'intermédiaire du revêtement métallique structuré.

13. Elément constitutif de passage selon la revendication 12, dans lequel la structure rapportée présente également des régions dans lesquelles de la matière du corps de base de passage a été enlevée en surface.

14. Elément constitutif de passage selon la revendication 12 ou 13, dans lequel, dans des régions de bordure du revêtement métallique, leur épaisseur a été augmentée par rapport à des régions centrales ou a été diminuée avec des gradients de profils prédéfinis et/ou a été pourvue d'un contour prédéfini.
